# EUROPEAN PATENT SPECIFICATION

(11) **EP 3 922 917 B1**
(45) Date of publication and mention of the grant of the patent: **21.08.2024**
(21) Application number: 21178728.8
(22) Date of filing: 10.06.2021
(51) Int. Cl.: F24F 8/22, F24F 7/02, A61L 9/20, F24F 13/06

(54) **APPARATUS FOR CONTRASTING BACTERIAL AND/OR VIRAL CONTAMINATION IN A CLOSED ENVIRONMENT**
APPARAT ZUR BEGRENZUNG VON BAKTERIELLER UND/ODER VIRALER KONTAMINATION IN EINER GESCHLOSSENEN UMGEBUNG
APPAREIL POUR CONTRASTER LA CONTAMINATION BACTÉRIENNE ET/OU VIRALE DANS UN ENVIRONNEMENT FERMÉ

(30) Priority: 10.06.2020 IT 202000013888
(43) Date of publication of application: 15.12.2021
(73) Proprietor: Puricraft S.r.l., 31013 Codogne (TV) (IT)
(72) Inventor: CARLET, Michele, 31013 Codognè TV (IT)
(74) Representative: Soranzo, Benedetta

(56) References cited:
- WO-A1-2012/068569
- US-A1- 2019 219 284
- US-A1- 2019 292 315

## Description

### TECHNICAL FIELD

This teaching refers in general to an apparatus for contrasting bacterial and/or viral contamination in a closed environment, such as in lifts, fitting rooms in clothing shops, revolving security doors for access to banking and similar environments, public transport vehicles such as buses, trains, subways, public toilets, changing rooms or medical surgeries.

### BACKGROUND ART

The adoption of protocols and technical means to prevent contagion has become a priority nowadays, especially in view of the Covid-19 viral pandemic.

Two lines of action are generally taken today. A first line of action is to contrast infectious agents being passed from one individual to another; a second line of action is to sanitize environments so as to compromise the viability of infectious agents.

A traditional solution among the sanitization techniques known and available today is irradiation with UVC radiation and another is treatment with ozone. Both of these techniques have an action intensity and effectiveness speed which are compatible with the most widespread sanitization requirements. However, they are incompatible with the treatment of environments occupied by people because of the harmful effect on the human body by the UVC radiation and a dispersion of ozone that is asphyxiating in the concentrations necessary for sanitization, as well as the ability to generate serious irritation to the eyes, nose, throat and respiratory system in general.

Therefore, the solutions adopted to date in environments occupied by people take the form of social distancing and the use of masks that limit the spread of saliva droplets emitted into the environment during breathing or speaking, these being the primary vectors of viral Covid-19 agents.

However, the solution of social distancing in particular implies a significant reduction in the usability of cramped or in any case small spaces, such as small shops, lifts, public transport and the like.

Moreover, sanitizing the environment is a major issue, especially in high-traffic and mixed-use environments, such as public toilets, lifts, changing rooms and fitting rooms in garment shops, particularly in shopping centres.

The effect of contamination of surfaces and the presence in the air of microdroplets capable of harbouring viruses and bacteria is considerable in such environments.

Examples of apparatuses for sanitizing a closed environment are provided in the following prior art documents: WO2012/068569 A1, US2019/219284 A1, US2019/292315 A1.

### SUMMARY

The problem at the basis of the present invention is therefore that of contrasting viral and/or bacterial contamination while in particular allowing to increase the safe use of closed environments, and especially of small extensions and/or high-traffic and mixed-use environments, while requiring less social distancing in order to obtain an equal level of safety of the people occupying them at the same time and/or reducing the risk of contamination of the occupants by inhaling pathogens and/or contact with environmental surfaces.

The task of the present teaching is therefore to make available an apparatus for contrasting bacterial and/or viral contamination in a closed environment that makes it possible to reduce the social distancing necessary to maintain an appreciable degree of health safety for the people occupying it.

In the context of this task, one of the aims of the present teaching is to make available an apparatus for contrasting bacterial and/or viral contamination in a closed environment that enables containing the infectious load, whether of bacterial or viral nature, that may be present in such environments, especially deposited on surfaces or diffused in the air.

Another task of the present teaching is to make available an apparatus for contrasting bacterial and/or viral contamination in a closed environment that allows to sanitize the air of said environments, thus obtaining a significant reduction of the infectious load possibly present in the air of such environments. Another aim of the present teaching is to propose an apparatus for contrasting bacterial and/or viral contamination in a closed environment that does not require significant flows of air change for such environments.

This task, as well as these and other aims which will emerge more fully below, are attained by an apparatus for contrasting bacterial and/or viral contamination in a closed environment according to the accompanying claim 1.

Detailed features of an apparatus for contrasting bacterial and/or viral contamination in a closed environment according to the invention are given in the dependent claims.

Further features and advantages of the invention will emerge more fully from the description of a preferred but not exclusive embodiment of an apparatus for contrasting bacterial and/or viral contamination in a closed environment according to the invention, illustrated by way of at least one non-limiting example thereof in the accompanying drawings listed below.

### BRIEF DESCRIPTION OF THE DRAWINGS

Figure 1 is a perspective view of a simplified diagram of an apparatus for contrasting bacterial and/or viral contamination in a closed environment according to the present finding;
Figure 2 is the apparatus in Figure 1, in a different functional configuration;
Figures 3a, 3b and 3c are a variant of the apparatus in Figures 1 and 2, in three different functional configurations;
Figure 4 is a diagram of a detail of the apparatus in Figures 1 and 2 or 3a, 3b and 3c, relating to the suction means.

### DETAILED DESCRIPTION

With particular reference to the above-mentioned figures, an apparatus for contrasting bacterial and/or viral contamination in a closed environment A is indicated in its entirety by 10, and comprises:
- a wall 11 configured to be incorporated in a ceiling or to constitute a ceiling of an environment A which may be occupied by people 100, so as to delimit said environment A; said wall 11 having a plurality of operating portions 12 which are open or permeable to an airflow to allow air to pass through the operating portions 12;
- suction means 13 connected to the wall 11 to suck air through the operating portions 12.

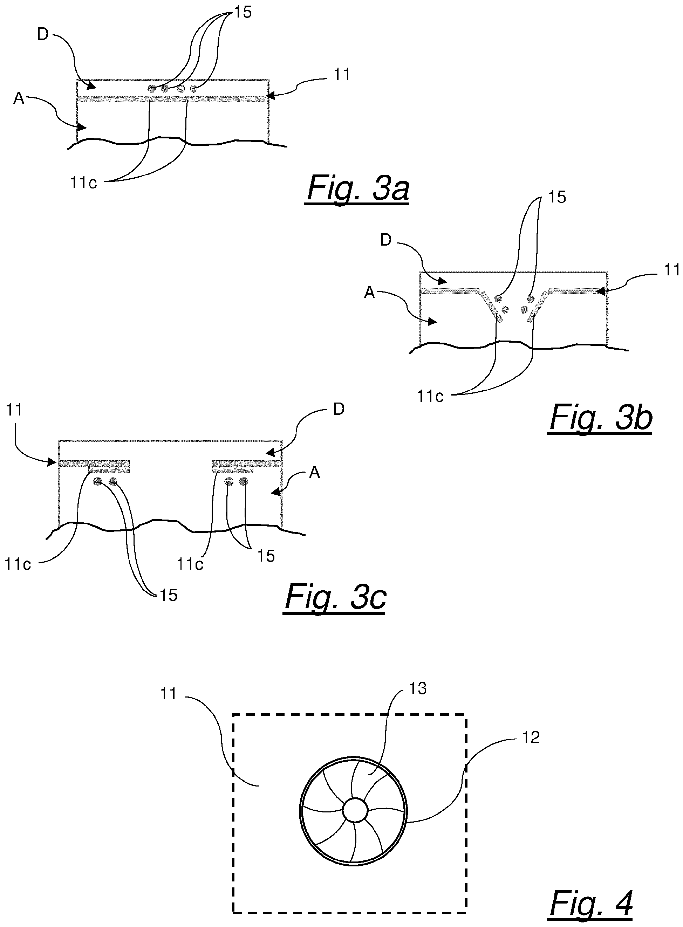

The operating portions 12 may comprise or consist of holes, for example circular holes, as by way of non-limiting example depicted in Figure 4.

The suction means 13 may comprise or consist of fans, for example axial fans. By way of non-limiting example, the suction means 13 have been schematised in Figure 4 as an axial fan engaging an operating portion 12.

The suction means 13 are configured to form, following their actuation, an updraft directed towards the wall 11 having a flow rate which, expressed in m³/h per unit of surface, that is (m³/h)/m², may be between 60 and 3000, and in particular between 120 and 1500, for example between 200 and 350, possibly between 230 and 300, for example it may be equal to 265 in an optimal solution.

In this way, when the suction means 13 are active, an upward airflow directed towards the wall 11 is formed in the environment A, whereby the saliva droplets emitted - during breathing or during speaking - by a first person of people 100 in the environment A, are dragged towards the wall 11 and, through the latter, directed outside the environment A before they can be inhaled by a second person present in the environment A.

This makes it possible to prevent bacterial and/or viral contamination between the people in the environment A even if the environment is crowded, i.e. there are people present in it at a shorter distance from each other than the safety distance necessary to prevent direct contagion in the event of an airborne disease.

The suction means 13 may be configured to give said updraft an average speed which is not less than 2 cm/s, possibly not less than 5 cm/s and, for example between 7 and 8 cm/s, and preferably which is not greater than 25 cm/s, in order to obtain an advantageous compromise between the performance required of the suction means and the effectiveness of the suction effect.

For example, with suction means 13 configured to determine an average speed of said updraft equal to 26400 cm/h, i.e. 7.3 cm/s, considering an average breathing frequency of 0.3 breaths/s, an average upward path of 24.4 cm of the saliva droplets emitted during a breath is obtained in the time between two breaths.

Thus, such saliva droplets emitted by a first person cannot reach the vicinity of the head of a second person adjacent to the first person in time to be inhaled in by the second person as a result of breathing, particularly if the first person is wearing a surgical mask or similar means of protection.

For example, an average speed of the updraft of between 7 and 8 cm/s may be obtained by providing suction means 13 consisting of fans housed in the holes of the operating portions 12, where these holes face a floor area surface of the environment A of about 3 m², for example 2.8 m² as in the case of a lift, where said fans have a nominal flow rate of 741 m³/h.

The suction means 13 may be configured to generate a flow rate of said updraft having a ratio with respect to an average flow rate generated by a person's breath which is greater than 20, possibly greater than 50, and for example greater than 80 and preferably less than 250.

The suction means 13 may be configured to generate a flow rate of said updraft between 300 l/min and 8000 l/min, and preferably between 600 l/min and 750 l/min per person, with respect to a nominal capacity of people of the environment A.

The suction means 13 are configured to generate a flow rate of said updraft having a ratio of flow rate volume per hour with respect to the floor area of the environment A between 60 m³/h and 3000 m³/h per m² of floor area.

Thus, estimating an average flow rate of breath generated by a person of approximately 8 l/min, the ratio of the updraft flow rate to the average flow rate of breath generated by a person may be approximately between 75 and 90. The suction means 13 and/or the operating portions 12 are configured to define, for each operating portion 12, an operating direction B along which air is sucked through the operating portion 12 following an actuation of the suction means 13. The operating directions B of each operating portion 12 are mutually parallel and parallel to a main direction C of the air stream.

The suction means 13 and/or the operating portions 12 are configured in such a way that following actuation of the suction means 13, the air forming said air stream has substantially uniform directions and speeds in at least one plane perpendicular to the main direction C so that said updraft is substantially uniform in order to avoid air mixing which could limit the dragging effect of saliva droplets of people present in the environment.

The operating portions 12 may be evenly distributed on the wall 11 to promote uniformity of said updraft.

As anticipated, each of the operating portions 12 may structurally comprise a hole through the wall 11.

The suction means 13 may comprise a plurality of fans each inserted in the hole or in fluidic communication with the hole to suck air through the hole.

The suction means 13 define a suction face 11a and a delivery face 11b of the wall 11.

In particular, the suction face 11a may face the environment A and the delivery face 11b may be opposite to the suction face 11a.

The apparatus 10 comprises:
- a casing 14 which, in cooperation with the delivery face 11b, delimits a treatment compartment D; wherein the suction means 13 are configured to introduce, into the treatment compartment D, air sucked in through the operating portions 12;
- sanitizing means 15 which are placed in the treatment compartment D or in fluidic communication with said treatment compartment D and are configured to reduce a bacterial and/or viral load present in the air which faces and/or laps said sanitizing means 15.

The casing 14 may be configured to form a drawer or suspended ceiling to be attached to the ceiling and/or walls of an environment A to be sanitized.

In particular, depending on the structure in which they are to be incorporated, the wall 11 and the casing 14 may be configured and sized in such a way that, in operation, the action of the suction means 13 combined with the distribution of the operating portions 12 on the wall 11 is such as to obtain an updraft effect in the entire environment A that the apparatus 10 is intended to sanitize in the context of the specific contingent application.

The sanitizing means 15 are configured to irradiate the interior of the treatment compartment D with UV-C radiation having an irradiation per unit of time which is not less than 1300 microW/cm², which can be higher than or equal to 12000 microW/cm², for example equal to about 14000 microW/cm² to obtain an optimal compromise between sanitization effectiveness and functionality of the apparatus 10.

This irradiation may be limited to values no greater than 22000 microW/cm². UV-C radiation is light radiation in the ultraviolet range with a wavelength which may be between 100 nm and 280 nm.

In particular, the sanitizing means 15 may comprise or consist of at least one lamp capable of emitting UV-C radiation.

The number of these lamps and/or their distribution may be chosen according to the contingent requirements for implementing this teaching in order to obtain a complete and effective irradiation of the air sucked from environment A into the treatment compartment D.

The treatment compartment D and the sanitizing means 15, which comprise or consist of first UV-C radiation emitters, are chosen to result in an irradiation time of at least 0.5 s and which may be greater than 1.5 s, for example equal to about 3 s for an optimal compromise between sanitization effectiveness and functionality of the apparatus 10, and preferably not greater than 5 s.

In other words, the air flow rate through the treatment compartment D, the shape of the treatment compartment, and the arrangement and configuration of the sanitizing means 15, such as the UV-C radiation emitter, are chosen in such a way that microorganisms carried by this air flow rate are subjected to irradiation for no less than 0.5 s.

This irradiation time may be determined by the action of the suction means 13 which may cause an airflow, sucked in from the environment A, to pass through the treatment compartment D.

The apparatus 10 may comprise at least one discharge passageway 16 capable of placing the treatment compartment D in fluidic communication with the environment A in order to allow the air sucked in and treated in the treatment compartment D to return to the environment.

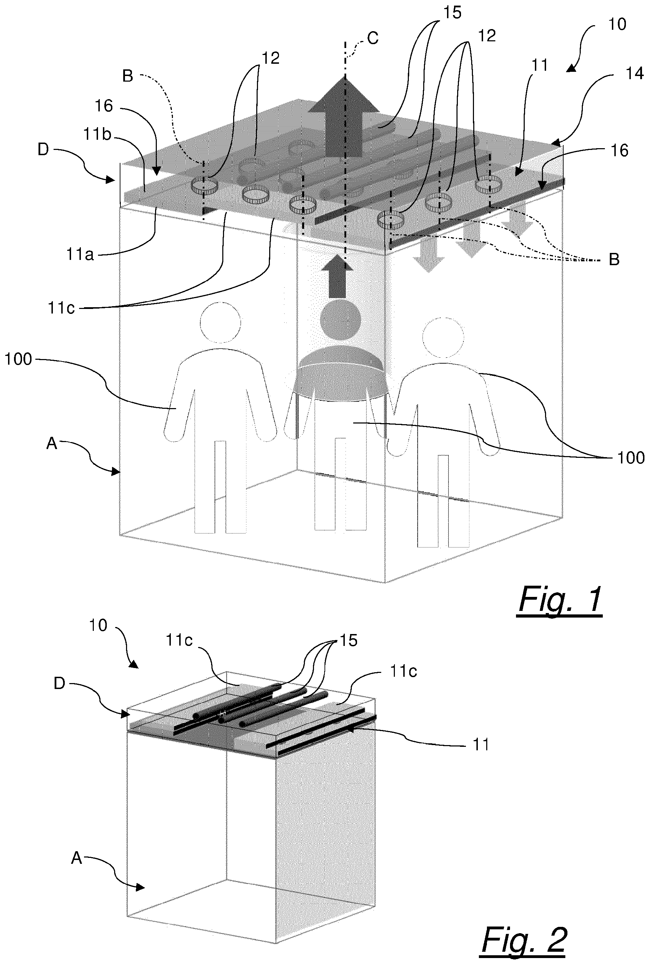

The discharge passageway 16 may be adapted to place the treatment compartment D in fluidic communication with a space in front of the suction face 11a, as illustrated by way of non-limiting example in Figure 1.

The discharge passageway 16 may comprise or consist of an opening or a slot or a plurality of slots crossing/passing through at least one perimeter edge of the wall 11, as far as possible from the operating portions 12 so as not to interfere with the updraft generated by suction through the latter.

The discharge passageway 16 may comprise at least one duct (not illustrated) extending from the wall 11, for example from at least one of its perimeter edges, and having at least one discharge opening intended to face the environment below the wall 11.

In an embodiment of the present teaching, such a duct, which may be part of a plurality of ducts, may be configured to have at least one discharge opening which, when positioned in environment A, is proximal to a floor of said environment A.

In this way, an upward motion of the air present in said environment A will be further promoted due to the effect both of the suction of the suction means 13, from the wall 11, and the thrust of the air introduced through the duct near the floor of the environment A.

The wall 11 may be configured to be open to sanitize the environment A, both the air in it and its surfaces, and/or to facilitate the maintenance of the apparatus 10.

The sanitizing means 15 may be positioned in such a way with respect to the wall 11 that following an opening of the wall 11, the sanitizing means 15 face into the environment A to treat its interior.

Structurally, the wall 11 may comprise at least one movable portion 11c having a supporting face.

The sanitizing means 15 comprise at least one sanitizing device, which may for example consist of at least one UV-C radiation emitting device, as mentioned above, attached to the supporting face of the movable portion 11a.

The movable portion 11c may be movable, for example by sliding - as exemplified in Figure 2 - and/or by rotation - as exemplified in Figures 3a, 3b and 3c - between a closed position and an open position.

The apparatus 10 may be configured in such a way that:
- in the closed position, e.g. visible in Figure 3a, the sanitizing device may be inside the treatment compartment D;
- in the open position, the sanitizing device may be external to the treatment compartment D in order to project into and/or face into environment A, as shown by way of non-limiting example in Figure 3c.

Figure 3b also shows a transition phase from the closed position to the open position.

By modulating the opening, i.e. the transition between the open position and the closed position, it is possible to adjust the orientation of the sanitizing means 15 which are attached to each movable portion 11c, and thus the extent and direction of treatment, e.g. of irradiation with UV-C radiation, of the internal surfaces of the environment A.

In one of the possible embodiments of the present teaching in accordance with which the wall 11 is openable to allow a sanitization of the environment A by the sanitization means 15, the latter may be configured to emit ultraviolet UV-C radiation having an irradiation per unit of time which is not less than 5 mW/cm², and which may be about 100 mW/cm², for example in the case of a distance between the sanitizing means 15 and a surface to be sanitized, for example the floor, of the environment A substantially not greater than 2.2 m and not greater than, for example, 130 mW/cm².

In such a case for example, a sanitization treatment of the environment A by irradiation using the sanitizing means 15 as specified above may be optimal following continuous irradiation for at least 811 s or 13.5 min.

Clearly, the duration of irradiation for sanitization and the intensity of the radiation may be chosen according to the specific configuration of the environment A to be sanitized and/or the degree of sanitization required and/or the degree of contamination of the environment A.

The sanitizing means 15 may comprise or consist of second emitters of UV-C radiation at a wavelength of about 220 nm, with the advantage that such radiation has been found not to be harmful to the human body, and/or third emitters of a radiation in the visible spectrum with a wavelength between about 406 nm and 413 nm (deep blue).

Such second and/or third emitters may be incorporated in lighting elements capable of illuminating the environment A and which may be attached to the wall 11 so as to face and/or project into the environment A itself.

In this case, the action of these emitters may be associated with a containment of bacterial and/or microbiological proliferation rather than with a sanitization of the environment itself, so they may advantageously be combined with the first emitters to combine an action of containment of microbiological and bacterial proliferation and sanitization of the environment A.

The apparatus 10 may also comprise a surface covering of the environment A comprising titanium dioxide TiO2 which may for example, be incorporated in a paint for covering surfaces, for example the side walls, of the environment A in order to obtain a contrasting effect to the bacterial proliferation and, in the case of adoption of the second and/or third emitters, to assist their effect also of eliminating odours in the environment A.

The apparatus 10 may comprise a control device (not shown), which may be connected to the suction means 13 and/or the sanitizing means 15 to operate them on command.

The apparatus 10 may also comprise first sensors directly or indirectly connected to the wall 11 to face, in operation, the environment A and configured to detect a presence of a person in the environment A.

The first sensors may for example, comprise or consist of:
- at least one presence sensor;
- at least one motion sensor;
- at least one proximity sensor, e.g. capacitive;
- at least one temperature sensor.

At least two first sensors may be chosen, e.g. from those listed above, configured to be redundant in order to ensure a high level of safety of the apparatus 10. Furthermore, the apparatus 10 may comprise second sensors directly or indirectly connected to the part 11 to face, in operation, the environment A and configured to detect values of environmental parameters of the environment A, chosen for example, from among:
- at least one ambient temperature sensor;
- at least one humidity sensor;
- at least one carbon dioxide presence and/or concentration sensor;
- at least one ozone presence and/or concentration sensor;
- at least one organic volatile compound presence and/or concentration sensor.

The apparatus 10 may comprise means for moving the at least one movable portion 11c of the wall 11, which may comprise or consist of for example, at least one linear actuator, which may be mechanical, for example a rack and pinion or ball screw, or hydraulic or pneumatic actuator.

The control device may be connected to the movement means of each movable portion 11c and configured to actuate them.

In addition, the control device may be configured to control the movement means to prevent the wall 11 from opening if the sanitizing means 15 are active, for example if the first emitters are active.

Furthermore, the apparatus 10 may comprise a telecommunications device such as a wireless and/or an interface device to enable interaction with an operator.

The control device may be connected to the telecommunications device and configured to communicate with a remote service via the telecommunications device to exchange operating data and/or parameters of the apparatus 10 detected by the first and/or second sensors.

The control device may also be configured to interface, via the telecommunications device, with a portable electronic device, such as a smartphone or similar, provided with a communication application configured to allow an operator to remotely control the control device via said portable electronic device.

The control device may be configured to actuate, following the opening of the wall 11, the sanitizing means 15 to emit UV-C ultraviolet radiation having an irradiation per unit of time which is not less than 5 mW/cm², and which may be about 100 mW/cm² and not more than e.g. 130 mW/cm².

In addition, the control device may be configured to actuate the sanitizing means 15 in order to actuate a sanitization of the surfaces of the environment A by irradiation lasting not less than 1 min and preferably not more than 660 min. It is therefore clear how the present teaching enables the set tasks and aims to be achieved by making available an apparatus 10 for contrasting bacterial and/or viral contamination in a closed environment A that makes it possible to reduce the social distancing necessary to maintain an appreciable degree of health safety for the people occupying it.

In fact, the suction action implemented by the suction means 13 through the operating portions 12 results in an updraft that prevents the transfer of saliva droplets resulting from breathing and/or speaking, thus generating an upward dragging of the same such that they are unlikely to be transferred between two people occupying the environment A monitored by the apparatus 10.

The apparatus 10 allows the containment of the infectious load - be it bacterial or viral - possibly present in such environments through the action of the second and third emitters and possibly also through the sanitizing action carried out by the first emitters on the air first sucked from the environment A and then reintroduced therein following sanitization.

Furthermore, an apparatus 10 allows to sanitize the air and the surfaces of an environment A, thus obtaining a considerable reduction of the infective load that may be present in the air of such an environment even without requiring significant flows of air change, rather by sanitizing and re-introducing the air present in the environment itself.

The invention thus conceived is susceptible to numerous modifications and variations if these fall within the scope of protection of the attached claims.

Where the operating and technical features mentioned in the following claims are followed by signs or reference numbers, the signs or reference numbers have been used only with the aim of increasing the intelligibility of the claims themselves and, consequently, they do not constitute in any way a limitation to the interpretation of each element identified, purely by way of example, by the signs or reference numerals.

In particular, further embodiments may comprise the technical features of one of the following claims with the addition of one or more described and/or illustrated technical features, taken individually or in any mutual combination.

## Claims

1. Apparatus (10) for contrasting bacterial and/or viral contamination in a closed environment (A), comprising:
- a wall (11) configured to be incorporated in a ceiling or to constitute a ceiling of an environment (A) which may be occupied by people (100), so as to delimit said environment (A); said wall (11) having a plurality of operating portions (12) which are open or permeable to an airflow to allow air to pass through said operating portions (12);
- suction means (13) connected to said wall (11) to suck air through said operating portions (12);
wherein said suction means (13) are configured to form, following their actuation, an updraft directed towards said wall (11) having a flow rate which, expressed in m³/h per unit of surface of the floor area of said environment (A) expressed in m², is between 60 and 3000;
wherein said suction means (13) and/or said operating portions (12) are configured to define, for each operating portion (12), an operating direction (B) along which air is sucked through said operating portion (12) following an actuation of said suction means (13); wherein the operating directions (B) of each operating portion (12) are mutually parallel and parallel to a main direction (C) of said air stream formed by said updraft;
wherein said suction means (13) and/or said operating portions (12) are configured such that following an actuation of said suction means (13), the air forming said air stream has substantially uniform directions and speeds in at least one plane perpendicular to said main direction (C);
wherein said suction means (13) define a suction face (11a) and a delivery face (11b) of said wall (11); said apparatus (10) comprising:
- a casing (14) which, in cooperation with said delivery face (11b), delimits a treatment compartment (D); said suction means (13) being configured to introduce, into said treatment compartment (D), air sucked in through said operating portions (12);
- sanitizing means (15) which are placed in said treatment compartment (D) or in fluidic communication with said treatment compartment (D) and are configured to reduce a bacterial and/or viral load present in the air which faces and/or laps said sanitizing means (15);
wherein said sanitizing means (15) are configured to irradiate the interior of said treatment compartment (D) with a UV-C radiation having an irradiation per unit of time which is not less than 1300 microW/cm²;
wherein the treatment compartment (D) and the sanitizing means (15) are chosen to result in an irradiation time of at least 0.5 s.

2. Apparatus (10) according to claim 1, wherein said operating portions (12) are uniformly distributed on said wall (11).

3. Apparatus (10) according to any one of the preceding claims, wherein each of said operating portions (12) comprises a hole passing through said wall (11); said suction means (13) comprise a plurality of fans each inserted in said hole or in fluidic communication with said hole and configured to suck air through said hole

4. Apparatus (10) according to any one of the previous claims, comprising at least one discharge passageway (16) placing said treatment compartment (D) in fluidic communication with said environment (A), and adapted for introducing treated air into said treatment compartment (D).

5. Apparatus (10) according to any one of the previous claims, wherein said wall (11) is configured to be opened, said sanitizing means (15) being positioned in such a way with respect to said wall (11) that following an opening of said wall (11), said sanitizing means (15) face said environment (A) to treat the interior of said environment (A).

6. Apparatus (10) according to claim 5, wherein said wall (11) comprises at least one movable portion (11c) having a supporting face; wherein said sanitizing means (15) comprise at least one sanitizing device attached to said supporting face; said movable portion (11c) being movable between a closed position and an open position, wherein:
- in said closed position, said sanitizing device is inside said treatment compartment (D);
- in said open position, said sanitizing device is external to said treatment compartment (D) to project into and/or face said environment (A).

## Patentansprüche

1. Einrichtung (10) zum Kontrastieren bakterieller und/oder viraler Kontamination in einer geschlossenen Umgebung (A), umfassend:
- eine Wand (11), die konfiguriert ist, um in eine Decke integriert zu werden oder eine Decke einer Umgebung (A) zu bilden, die von Menschen (100) in Anspruch genommen werden kann, um die Umgebung (A) abzugrenzen; wobei die Wand (11) eine Vielzahl von Betriebsabschnitten (12) aufweist, die für einen Luftstrom offen oder durchlässig sind, um zu ermöglichen, dass Luft durch die Betriebsabschnitte (12) hindurch strömt;
- Saugmittel (13), die mit der Wand (11) verbunden sind, um Luft durch die Betriebsabschnitte (12) hindurch zu saugen;
wobei die Saugmittel (13) konfiguriert sind, um, nach ihrer Betätigung, einen Aufwind zu erzeugen, der zu der Wand (11) hin gerichtet ist, der eine Strömungsrate aufweist, die, ausgedrückt in m³/h pro Oberflächeneinheit der Bodenfläche der Umgebung (A), ausgedrückt in m², zwischen 60 und 3000 liegt;
wobei die Saugmittel (13) und/oder die Betriebsabschnitte (12) konfiguriert sind, um, für jeden Betriebsabschnitt (12), eine Betriebsrichtung (B) zu definieren, entlang derer nach einer Betätigung der Saugmittel (13) Luft durch den Betriebsabschnitt (12) hindurch gesaugt wird; wobei die Betriebsrichtungen (B) jedes Betriebsabschnitts (12) zueinander parallel und parallel zu einer Hauptrichtung (C) des Luftstroms verlaufen, der durch den Aufwind ausgebildet wird;
wobei die Saugmittel (13) und/oder die Betriebsabschnitte (12) derart konfiguriert sind, dass, nach einer Betätigung der Saugmittel (13) die Luft, die den Luftstrom ausbildet, in mindestens einer Ebene senkrecht zu der Hauptrichtung (C) im Wesentlichen gleichmäßige Richtungen und Geschwindigkeiten aufweist;
wobei die Saugmittel (13) eine Saugfläche (11a) und eine Abgabefläche (11b) der Wand (11) definieren; die Einrichtung (10) umfassend:
- ein Gehäuse (14), das, im Zusammenwirken mit der Abgabefläche (11b) einen Behandlungsabteil (D) abgrenzt; wobei die Saugmittel (13) konfiguriert sind, um, in das Behandlungsabteil (D), Luft einzuleiten, die durch die Betriebsabschnitte (12) hindurch gesaugt wird;
- Desinfektionsmittel (15), die in dem Behandlungsabteil (D) oder in Fluidkommunikation mit dem Behandlungsabteil (D) platziert sind und konfiguriert sind, um eine bakterielle und/oder virale Belastung zu verringern, die in der Luft vorhanden ist, die den Desinfektionsmitteln (15) zugewandt ist und/oder sie umgibt;
wobei die Desinfektionsmittel (15) konfiguriert sind, um das Innere des Behandlungsabteils (D) mit einer UV-C-Strahlung zu bestrahlen, die eine Bestrahlungsleistung pro Zeiteinheit von nicht weniger als 1300 Mikrowatt/cm² aufweist;
wobei das Behandlungsabteil (D) und die Desinfektionsmittel (15) gewählt sind, um eine Bestrahlungszeit von mindestens 0,5 s zu ergeben.

2. Einrichtung (10) nach Anspruch 1, wobei die Betriebsabschnitte (12) gleichmäßig auf der Wand (11) verteilt sind.

3. Einrichtung (10) nach einem der vorstehenden Ansprüche, wobei jeder der Betriebsabschnitte (12) ein Loch umfasst, das durch die Wand (11) hindurch verläuft; wobei die Saugmittel (13) eine Vielzahl von Entlüftungsöffnungen umfassen, die jeweils in dem Loch oder in Fluidkommunikation mit dem Loch eingefügt und konfiguriert ist, um Luft durch das Loch hindurch zu saugen.

4. Einrichtung (10) nach einem der vorstehenden Ansprüche, umfassend mindestens einen Abflusskanal (16), der das Behandlungsabteil (D) in Fluidkommunikation mit der Umgebung (A) platziert und zum Einleiten behandelter Luft in das Behandlungsabteil (D) geeignet ist.

5. Einrichtung (10) nach einem der vorstehenden Ansprüche, wobei die Wand (11) konfiguriert ist, um geöffnet zu werden, wobei die Desinfektionsmittel (15) hinsichtlich der Wand (11) derart positioniert sind, dass, nach einem Öffnen der Wand (11), die Desinfektionsmittel (15) der Umgebung (A) zugewandt sind, um das Innere der Umgebung (A) zu behandeln.

6. Einrichtung (10) nach Anspruch 5, wobei die Wand (11) mindestens einen bewegbaren Abschnitt (11c) umfasst, der eine Stützfläche aufweist; wobei die Desinfektionsmittel (15) mindestens eine Desinfektionsvorrichtung umfassen, die an der Stützfläche angebracht sind; wobei der bewegbare Abschnitt (11c) zwischen einer geschlossenen Position und einer offenen Position bewegbar ist, wobei:
- in der geschlossenen Position, die Desinfektionsvorrichtung sich innerhalb des Behandlungsabteils (D) befindet;
- in der geöffneten Position, die Desinfektionsvorrichtung sich außerhalb des Behandlungsabteils (D) befindet und in die Umgebung (A) hinein ragt und/oder ihr zugewandt ist.

## Revendications

1. Appareil (10) permettant de contraster une contamination bactérienne et/ou virale dans un environnement fermé (A), comprenant :
- une paroi (11) conçue pour être incorporée dans un plafond ou pour constituer un plafond d'un environnement (A) qui peut être occupé par des personnes (100), de façon à délimiter ledit environnement (A) ; ladite paroi (11) ayant une pluralité de parties fonctionnelles (12) qui sont ouvertes ou perméables à un flux d'air pour permettre à de l'air de traverser lesdites parties fonctionnelles (12) ;
- des moyens d'aspiration (13) raccordés à ladite paroi (11) pour aspirer de l'air à travers lesdites parties fonctionnelles (12) ;
dans lequel lesdits moyens d'aspiration (13) sont conçus pour former, à la suite de leur actionnement, un courant ascendant dirigé vers ladite paroi (11) ayant un débit d'écoulement qui, exprimé en m³/h par unité de surface de l'aire de plancher dudit environnement (A) exprimée en m², est compris entre 60 et 3000 ;
dans lequel lesdits moyens d'aspiration (13) et/ou lesdites parties fonctionnelles (12) sont conçus pour définir, pour chaque partie fonctionnelle (12), une direction de fonctionnement (B) le long de laquelle de l'air est aspiré à travers ladite partie fonctionnelle (12) à la suite d'un actionnement desdits moyens d'aspiration (13) ; dans lequel les directions de fonctionnement (B) de chaque partie fonctionnelle (12) sont parallèles entre elles et parallèles à une direction principale (C) dudit courant d'air formé par ledit courant ascendant ;
dans lequel lesdits moyens d'aspiration (13) et/ou lesdites parties fonctionnelles (12) sont conçus de sorte qu'à la suite d'un actionnement desdits moyens d'aspiration (13), l'air formant ledit courant d'air a des directions et vitesses sensiblement uniformes dans au moins un plan perpendiculaire à ladite direction principale (C) ;
dans lequel lesdits moyens d'aspiration (13) définissent une face d'aspiration (11a) et une face de distribution (11b) de ladite paroi (11) ; ledit appareil (10) comprenant :
- un boîtier (14) qui, en coopération avec ladite face de distribution (11b), délimite un compartiment de traitement (D) ; lesdits moyens d'aspiration (13) étant conçus pour introduire, dans ledit compartiment de traitement (D), de l'air aspiré à travers lesdites parties fonctionnelles (12) ;
- des moyens de désinfection (15) qui sont placés dans ledit compartiment de traitement (D) ou en communication fluidique avec ledit compartiment de traitement (D) et sont conçus pour réduire une charge bactérienne et/ou virale présente dans l'air qui fait face auxdits, et/ou recouvre lesdits, moyens de désinfection (15) ;
dans lequel lesdits moyens de désinfection (15) sont conçus pour irradier l'intérieur dudit compartiment de traitement (D) avec un rayonnement UV-C ayant une irradiation par unité de temps qui n'est pas inférieure à 1300 microW/cm² ;
dans lequel le compartiment de traitement (D) et les moyens de désinfection (15) sont choisis pour résulter en un temps d'irradiation d'au moins 0,5 s.

2. Appareil (10) selon les revendications 1, dans lequel lesdites parties fonctionnelles (12) sont uniformément réparties sur ladite paroi (11).

3. Appareil (10) selon l'une quelconque des revendications précédentes, dans lequel chacune desdites parties fonctionnelles (12) comprend un trou traversant ladite paroi (11) ; lesdits moyens d'aspiration (13) comprennent une pluralité de ventilateurs insérés chacun dans ledit trou ou en communication fluidique avec ledit trou et conçus pour aspirer de l'air à travers ledit trou

4. Appareil (10) selon l'une quelconque des revendications précédentes, comprenant au moins un passage d'évacuation (16) plaçant ledit compartiment de traitement (D) en communication fluidique avec ledit environnement (A), et adapté pour introduire l'air traité vers ledit compartiment de traitement (D).

5. Appareil (10) selon l'une quelconque des revendications précédentes, dans lequel ladite paroi (11) est conçue pour être ouverte, lesdits moyens de désinfection (15) étant positionnés d'une manière telle par rapport à ladite paroi (11) qu'à la suite d'une ouverture de ladite paroi (11), lesdits moyens de désinfection (15) font face audit environnement (A) pour traiter l'intérieur dudit environnement (A).

6. Appareil (10) selon la revendication 5, dans lequel ladite paroi (11) comprend au moins une partie mobile (11c) ayant une face de support ; dans lequel lesdits moyens de désinfection (15) comprennent au moins un dispositif de désinfection fixé à ladite face de support ; ladite partie mobile (11c) étant mobile entre une position fermée et une position ouverte, dans lequel :
- dans ladite position fermée, ledit dispositif de désinfection est à l'intérieur dudit compartiment de traitement (D) ;
- dans ladite position ouverte, ledit dispositif de désinfection est externe audit compartiment de traitement (D) pour faire saillie dans ledit, et/ou faire face audit, environnement (A).
